# EUROPEAN PATENT APPLICATION

(11) **EP 4 349 837 A1**
(43) Date of publication of application: **10.04.2024**
(21) Application number: 22460049.4
(22) Date of filing: 05.10.2022
(51) Int. Cl.: C07D 473/04

(54) **A PHARMACEUTICAL INTERMEDIATE**

(71) Applicant: Zaklady Farmaceutyczne "Polpharma" S.A., 83-200 Starogard Gdanski (PL)
(72) Inventor: Grauzenis, Maciej, 83-200 Starogard Gdanski (PL); Sagol, Karol, 83-110 Tczew (PL); Haluszczuk, Adam, 80-058 Gdansk (PL)
(74) Representative: Elkington and Fife LLP

(57) **Abstract**

This invention relates to a quasi-amorphous form of (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate and a process to prepare this compound.

## Description

The present invention relates to a pharmaceutical intermediate, particularly (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, and a process for preparing (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

The present invention also relates to the preparation of 8-[(3R)-3-aminopiperidin-1-yl]-7-but-2-ynyl-3-methyl-1-[(4-methylquinazolin-2-yl)methyl]purine-2,6-dione (linagliptin) or a salt thereof using *(R)-tert-*Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

Linagliptin is named 8-[(3R)-3-aminopiperidin-1-yl]-7-but-2-ynyl-3-methyl-1-[(4-methylquinazolin-2-yl)methyl]purine-2,6-dione and has the following structure:

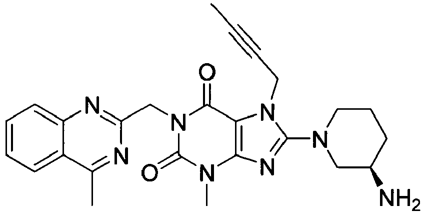

Linagliptin is a dipeptidyl peptidase 4 (DPP-4 inhibitor), and is classified as an oral hypoglycaemic agent.

It is marketed in a number of countries as Trajenta^{®}, an oral hypoglycaemic agent. It has been indicated for the treatment of type 2 diabetes mellitus as an adjunct to diet and exercise to improve glycaemic control as a monotherapy. It has also been indicated for the treatment of type 2 diabetes mellitus in combination with empagliflozin (Glyxambi^{®}) and in combination with metformin (Jentadueto^{®}).

Linagliptin may be prepared according to the procedures described in WO 2004018468 and WO 2014097314.

The process typically begins with a base-mediated condensation between a chloride compound (compound (I)) and a xanthine compound (compound (II)) to provide compound (III).

In the next step, the process includes a second base-mediated condensation between compound (III) and (*R*)-3-(Boc-amino)piperidine, to provide compound (V).

The tert-butyloxycarbonyl (Boc) protecting group is then removed to provide the target compound linagliptin.

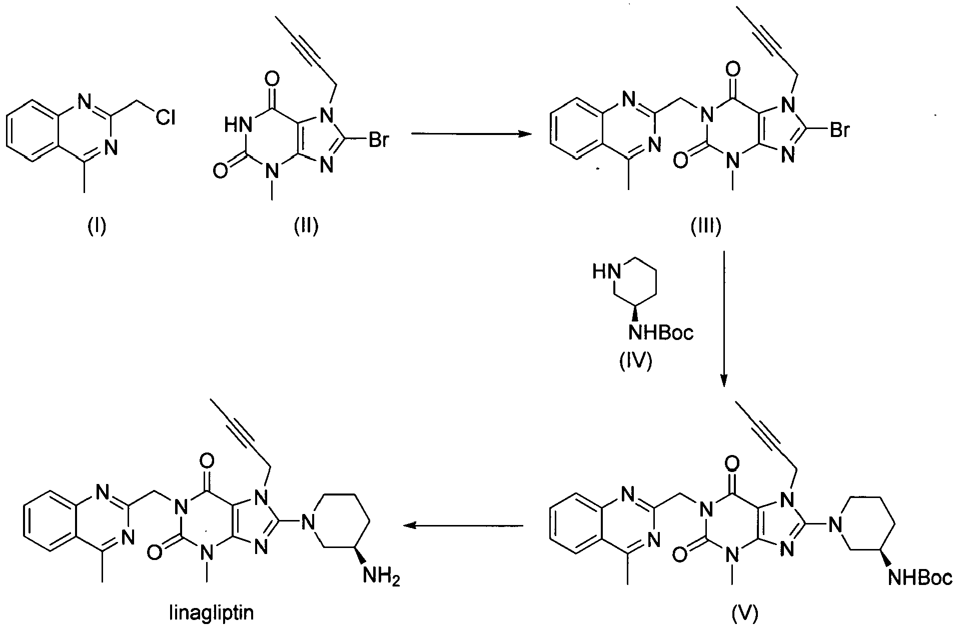

This process is well established in the art.

However, although this process provides linagliptin with an acceptable yield and purity, it requires the isolation of multiple intermediates prior to the isolation of the final product. Furthermore, complete removal of impurities is generally not possible using the processes described so far in the prior art and usually some coloured impurities remain in the final product. Accordingly, most commercially available linagliptin is not perfectly white, but has some yellowish coloration.

Consequently, further optimisation of the process is desirable.

Indeed, both large-scale chemical syntheses and purifications are subjected to constant review and optimisation by those skilled in the art. Owing to economies of scale, any small improvement in a given reaction or purification parameter is particularly economically significant. Therefore, optimisation of a large-scale synthesis or purification that provides, for example, an increase in chemical yield, decrease in step-to-step manipulation, decrease in reaction time, decrease in reaction temperature, decrease in amount of catalyst or solvent used, increase in the favourability of reagents, reduction in side-product formation, a more environmentally friendly synthesis or increase in chemical purity, is of interest both to chemical manufacturers and suppliers.

Moreover, step optimisation that reduces the need for multiple or hard-to-perform purifications are particularly beneficial. Any improvement in the ease of purification or isolation, through telescoping (wherein two or more, previously independent synthetic transformations converge into a "single" process and therefore require only one purification step) of synthetic procedures, or "one-pot" transformations, or the identification of an intermediate for recrystallisation, or precipitation, or removal of impurities through conversion into a transient intermediate, or substitution for a cheaper, more environmentally friendly or less toxic reagent, provide an attractive and economically desirable goal.

However, practical achievement of any such improvement is not straightforward.

For example, "one-pot" transformations (i.e. a reaction whereby a reactant is subjected to successive chemical reactions (a multistep synthesis) in a single reaction vessel) offer one attractive solution.

However, in practice, the lack of isolation of intermediates during a one-pot process typically leads to an accumulation of intermediate byproducts, which negatively affects product yield. Furthermore, intermediate byproducts that are structurally related to the target compound are often difficult to separate, which negatively affects product purity.

Typically, the removal of intermediate byproducts requires the use of expensive and time-consuming purification procedures, e.g. high-performance liquid chromatography (HPLC). However, such procedures are not economically feasible for large-scale transformations, where multiple kilogrammes of target compound are required.

In view of these challenges, various methods have been proposed to optimise the preparation of linagliptin.

One such method focuses on the manipulation of the polymorphic form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (compound (V)) as a means to selectivity isolate a key intermediate towards linagliptin whilst at the same time remove impurities.

For example, compound V is obtained in WO 2014097314 as an amorphous intermediate with high purity without the requirement for column chromatography. Equally, compound V is obtained in WO 2019064214, but as a crystalline intermediate, again without the requirement for column chromatography.

However, there are drawbacks associated with the procedures set out in WO 2014097314 and WO 2019064214.

First, although polymorphic forms of *(R)-tert-Butyl* (1-(7-(but-2-yn-1-yl)-3-methyl-1-((4-methylquinazolin-2-yl)methyl)-2,6-dioxo-2,3,6,7-tetrahydro-1H-purin-8-yl) are obtained without the requirement for column chromatography, they are obtained with sub-optimal purity.

Secondly, the processes require the isolation of multiple intermediates prior to the isolation of the final product, i.e. they do not offer a one-pot solution.

Consequently, because (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is a key intermediate to provide linagliptin, it would be highly desirable to provide a solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate using a simplified synthesis, with an increased purity and/or yield.

Ideally, the solid form would reduce or prevent the presence of impurities, such as compounds (I)-(IV) and by-products thereof, as well as any other impurities generated during the synthesis, in the final linagliptin product.

Consequently, there remains a need in the art for improved solid forms of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) (compound V).

Accordingly, the present invention provides a quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

The inventors have identified this solid form as quasi-amorphous because it displays long-range order and crystalline structure unlike material that would be considered to be in a fully amorphous state, however at the same time, the material does not possess all of the characteristics of a crystalline solid.

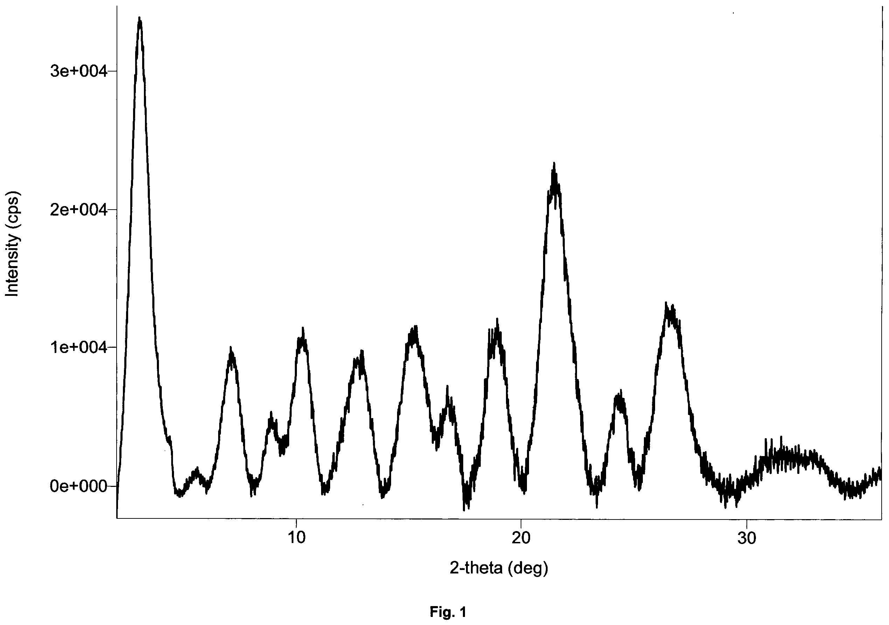

For example, the quasi-amorphous form of the present invention displays broad (as opposed to sharp) peaks in its powder X-ray diffraction spectrum (XRPD) (see Figure 1).

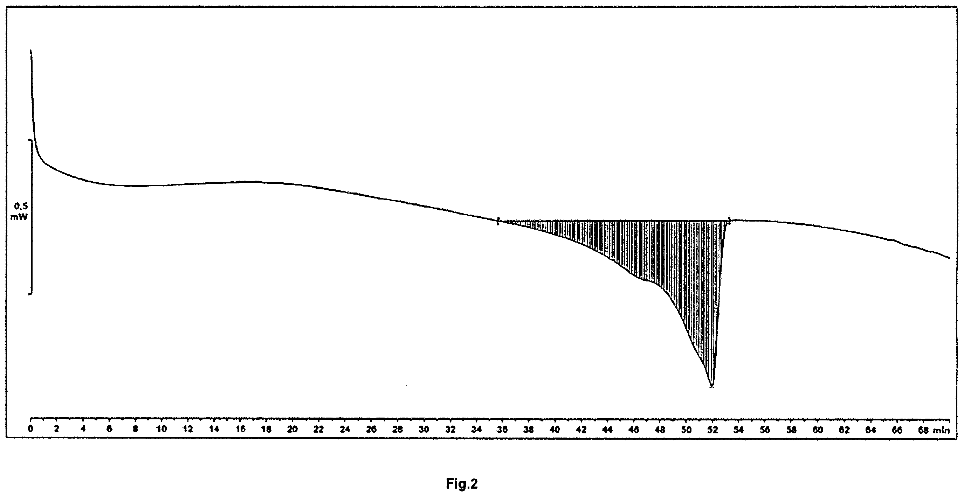

Furthermore, the differential scanning calorimetry (DSC) thermogram possesses a single sharp melting peak after an elongated melting endotherm (see Figure 2).

Pure crystalline material would exhibit only one sharp peak, with said peak corresponding to the melting of a specific polymorphic form. Equally, pure amorphous material would melt across a range with no particular end value, with an endotherm slowly appearing and fading.

However, as can be seen in Figure 2, the quasi-amorphous form displays a DSC thermogram having both crystalline and amorphous characteristics. In particular, the elongated endotherm indicates that the amorphous phase begins to melt first; following which the crystalline phase melts with a sharp peak.

As noted above, the quasi-amorphous form of the present invention displays broad (as opposed to sharp) peaks in its powder X-ray diffraction spectrum (XRPD). It is therefore possible to define the XRPD peaks with reference to the sharp point at the top of each broad peak, or with reference to the breadth of each peak calculated at the base of each peak.

Therefore, the present invention also provides a quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate characterised by XRPD peaks at 2θ (°) ± 0.2°: 3.0, 10.3, 15.3, 18.8, 21.4 and 26.5.

The characterising peaks, including those of lower relative intensity, are as follows.
XRPD 2θ (°) ± 0.2°: 3.0, 7.1, 8.9, 10.3, 12.7, 15.3, 16.8, 18.8, 21.4, 24.3 and 26.5.

The present invention also provides a quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate characterised by broad XRPD peaks, wherein the breadth of each peak, at the base of each peak is 2θ (°) ± 0.2°: 2.3-4.0, 9.7-10.7, 14.8-15.8, 20.5-22.5 and 25.8-27.5.

For the avoidance of any doubt, the breadth of each peak is calculated at the base of each peak by taking each 2θ (°) ± 0.2° value at the point at which the upslope of a peak originates from the x-axis, and at the point at which the downslope of a peak meets the x-axis of the XRPD.

In one embodiment, the quasi-amorphous form of *(R)-tert-Butyl* (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, may further be characterised by a differential scanning calorimetry (DSC) thermogram having a melting onset at 144-146°C, a melting endset at 156-158°C and a melting point peak at 154-156°C.

In particular, the quasi-amorphous form may further be characterised by a differential scanning calorimetry (DSC) thermogram having a melting onset at 144-146°C, a melting endset at 156-158°C and a melting point peak at 154.5-155.5°C.

In one embodiment, the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, may be characterised by the XRPD according to Figure 1.

In one embodiment, the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, may be characterised by the DSC thermogram according to Figure 2.

The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate of the present invention may also be characterised by the following characterising peaks.

IR (cm⁻¹): 596, 761, 954, 1054, 1129, 1167, 1245, 1288, 1311, 1365, 1401, 1439, 1515, 1569, 1621, 1664, 1701, 2942 and 3334.

The present invention also provides a process to prepare a solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate comprising:
(i) dissolving (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate in dichloromethane to form a solution;
(ii) adding methyl tert-butyl ether to the solution; and
(iii) collecting the resulting precipitate from the solution.

It has been found that this process provides a solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate in high yield and high purity without recourse to column chromatography.

The present invention will now be described with reference to the accompanying drawings, in which:
Fig. 1 shows the XRPD spectrum of the quasi-amorphous form of (*R*)-*tert*-*Butyl* (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (the product of Example 2);
Fig. 2 shows the DSC trace of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (the product of Example 2); and
Fig. 3 shows the IR trace of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (the product of Example 2);

In step (i) of the process, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution.

In one embodiment, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution, activated charcoal is then added to the solution to form a suspension, and the resulting suspension is allowed to stir.

In one embodiment, magnesium sulphate may also be added to the dichloromethane solution in addition to the activated charcoal.

The suspension may be stirred for a duration of between 1 to 3 hours. Preferably, the suspension is stirred for a duration of 2 hours.

Preferably, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution, and activated charcoal and magnesium sulphate are added to the solution, and the resulting mixture is allowed to stir for a duration of between 1 to 3 hours, preferably 2 hours, prior to step (ii).

If activated charcoal or activated charcoal and magnesium sulphate are added to the dichloromethane solution, they are removed prior to step (ii). Typically, the activated charcoal and magnesium sulphate are removed by filtration. Alkali activated charcoal is preferred.

The dichloromethane solution formed in step (i), typically, the resulting solution following the removal of the activated charcoal and magnesium sulphate, may be heated to between 20°C and 40°C, preferably between 25°C and 38°C, prior to addition of methyl tert-butyl ether in step (ii).

Preferably, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution, activated charcoal and magnesium sulphate are added to the solution, and the resulting mixture is allowed to stir for a duration of between 1 to 3 hours, preferably 2 hours. The activated charcoal and magnesium sulphate are then removed from the solution, and the solution is heated to between 20°C and 40°C, preferably between 25°C and 38°C, prior to addition of methyl tert-butyl ether in step (ii).

In one embodiment, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution, and the resulting solution is heated to between 20°C and 40°C, preferably between 25°C and 38°C, more preferably between 34°C and 36°C, prior to the addition of methyl tert-butyl ether in step (ii).

In step (ii) of the process, methyl tert-butyl ether is added to the dichloromethane solution, and the resulting mixture is allowed to stir.

Preferably methyl tert-butyl ether is added dropwise to the dichloromethane solution over a duration of between 10 minutes and to 2 hours, preferably over a duration of 1 hour.

Methyl tert-butyl ether is typically added such that upon complete addition of the methyl tert-butyl ether, the ratio between the volumes of dichloromethane to methyl tert-butyl ether is from 1:1 to 1:10. Preferably, the ratio between the volumes is from 1:2 to 1:8, even more preferably the ratio between the volumes is from 1:3 to 1:6, for example 1:4.

Upon complete addition of the methyl tert-butyl ether to the dichloromethane solution, the resulting mixture is cooled to between 0°C and 10°C, preferably to 5°C, preferably at a cooling rate of -10°C/hour.

Once cooled, the mixture may be stirred for a duration of between 1 to 3 hours, preferably 2 hours.

In one embodiment, in step (i) of the process, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dissolved in dichloromethane to form a solution, the solution is heated to between 20°C and 40°C, preferably between 25°C and 38°C, more preferably between 34°C and 36°C; and in step (ii) of the process, methyl tert-butyl ether is then added dropwise to the dichloromethane solution, and upon complete addition of the methyl tert-butyl ether, the resulting mixture is cooled to between 0°C and 10°C, preferably to 5°C, preferably at a cooling rate of -10°C/hour.

Preferably, activated charcoal is added to the solution, and the mixture stirred for a duration of between 1 to 3 hours, preferably 2 hours, before the activated charcoal is removed by filtration, prior to step (ii). Alkali activated charcoal is preferred.

Preferably, methyl tert-butyl ether is typically added such that upon complete addition of the methyl tert-butyl ether, the ratio between the volumes of dichloromethane to methyl tert-butyl ether is from 1:3 to 1:6, for example 1:4.

In step (iii) of the process, the resulting precipitate, the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, is collected from the mixture, preferably by gravity or vacuum filtration.

Before isolating the solid form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate, the precipitate may be washed with a mixture of dichloromethane and methyl tert-butyl ether (typically 1:4 dichloromethane:methyl tert-butyl ether).

Following this, the solid form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is dried, to provide a white solid with a high degree of purity.

Preferably, the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate that is provided by the process described hereinabove is the quasi-amorphous form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate provided by the process may be defined and characterised as described hereinabove.

Prior to the performance of steps (i)-(iii), *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate may be synthesised.

In one embodiment, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is prepared by reacting a compound of formula (III) with a compound of formula (IV), or a salt thereof: 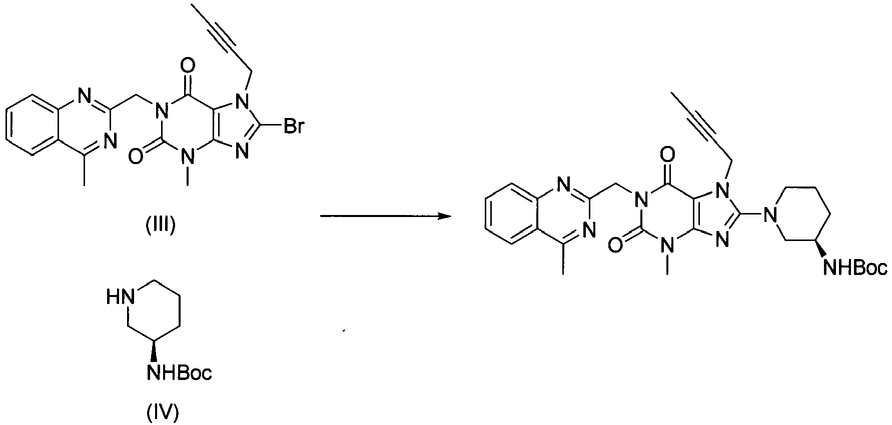 prior to steps (i) to (iii).

Typically, the compound of formula (III) is reacted with the compound of formula (IV) in the presence of a base, for example an inorganic base such as potassium carbonate (K₂CO₃).

The temperature of the reaction may vary depending upon the solvent used. Preferably, the reaction is conducted using a nitrogen-containing solvent as the reaction solvent, and the reaction is heated to a temperature of between 60°C and 70°C. Preferably, the nitrogen-containing solvent is N-methyl-2-pyrrolidone (NMP).

Under these conditions, complete conversion to (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is routinely achieved in 8 to 12 hours.

The compound of formula (III) may be prepared by reacting a compound of formula (I) with a compound of formula (II):

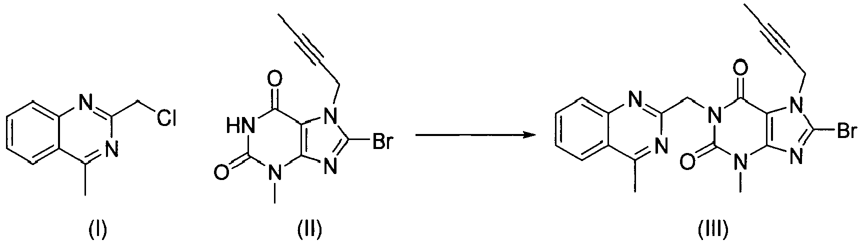

Typically, the compound of formula (I) is reacted with the compound of formula (II) in the presence of a base, for example an inorganic base such as potassium carbonate (KzCOs).

The temperature of the reaction may vary depending upon the solvent used. Preferably, the reaction is conducted using a nitrogen-containing solvent as the reaction solvent, and the reaction is heated to a temperature of between 70°C and 80°C.

The nitrogen-containing solvent is typically an *N*-alkylpyrrolidinone, dimethylformamide, or dimethylacetamide.

Preferably, the nitrogen-containing solvent is *N*-methyl-2-pyrrolidone (NMP).

Under these conditions, complete conversion to compound of formula (III) is routinely achieved in 2 to 6 hours.

The reaction may also be conducted in the presence of a catalyst and, preferably, in the presence of a catalyst and an antioxidant compound.

The catalyst is preferably an iodide salt, for example potassium iodide, sodium iodide, lithium iodide or ammonium iodide. Most preferably, the catalyst is potassium iodide.

It is understood that a catalyst, in particular an iodide salt advantageously increases the rate of reaction in the direction compound (III). However, it is also understood that iodide salts may react with the reaction solvent, in particular if the reaction solvent is a nitrogen-containing solvent, such as NMP. The inventors surprisingly discovered that if the reaction is conducted in the presence of an iodide salt and an antioxidant compound, then the reaction between the iodide salt and the nitrogen-containing solvent can be supressed or eliminated.

This is particularly advantageous, because the reaction is allowed to proceed in the presence of a catalyst and the formation of byproducts is reduced. This in turn further increases both reaction yield and the purity of the reaction product.

The antioxidant compound may be selected from butylated hydroxyanisole, butylated hydroxytoluene, methyl gallate, ethyl gallate, propyl gallate, gallic acid, butylated hydroquinone and phenol.

Preferably, the antioxidant compound is butylated hydroxyanisole.

In one embodiment, prior to the performance of steps (i)-(iii), (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate may be prepared according to the following process: 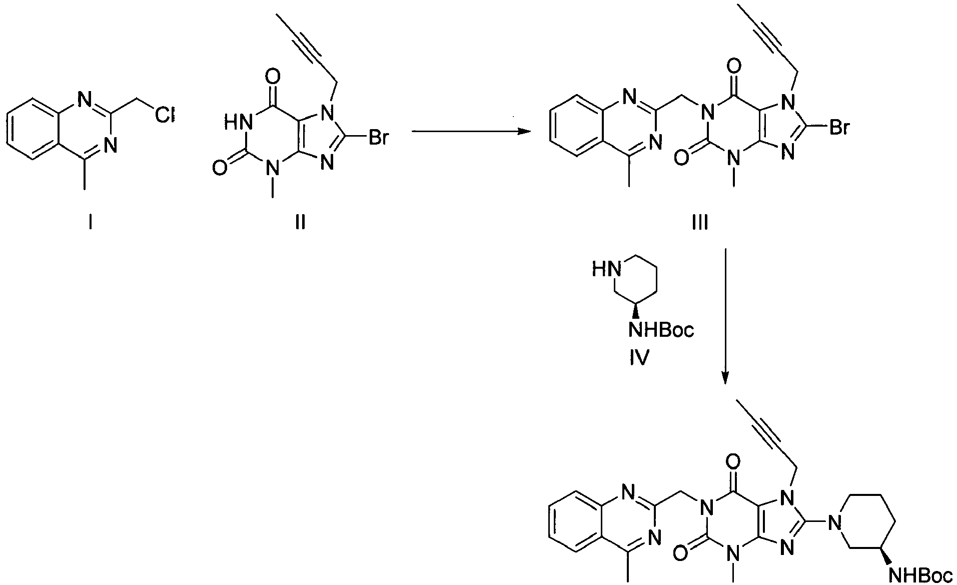 wherein the process is a one-pot process.

For the avoidance of any doubt, a one-pot process is a process/reaction whereby a reactant is subjected to successive chemical reactions (a multistep synthesis) in a single reaction vessel.

Advantageously, following this one-pot procedure and upon completion of process steps (i)-(iii) defined hereinabove, the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate may be isolated in high yield and high purity.

Thus, by preparing and isolating the solid form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as described, the inventors discovered that the reaction steps, including reacting together the compounds of formulae (I) and (II) to form the compound of formula (III), and reacting together the compounds of formulae (III) and (IV) to form (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate may be performed in one-pot.

In particular, by preparing and isolating the solid form according to the process defined herein, a one-pot process may be used. This is owing to the fact that the solid form can be obtained effectively in the absence of impurities and byproducts.

In particular, isolation of the solid form from the one pot process effectively removes any intermediate byproducts that are typically formed during the reaction of the compounds of formulae (I) and (II) to form the compound of formula (III).

Thus, the process of preparing the solid form renders the one-pot process feasible in terms of the overall yield and purity of the product obtained.

Preferably, the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate that is provided by the process described hereinabove is the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate provided by the process may be defined and characterised as described hereinabove.

The one-pot process may be conducted by using the synthetic procedures defined hereinabove.

In particular, the compound of formula (I) is reacted with the compound of formula (II) in the presence of a base, for example an inorganic base such as potassium carbonate (K₂CO₃).

The temperature of the reaction may vary depending upon the solvent used. Preferably, the reaction is conducted using a nitrogen-containing solvent as the reaction solvent, and the reaction is heated to a temperature of between 70°C and 80°C. Preferably, the nitrogen-containing solvent is N-methyl-2-pyrrolidone (NMP).

Under these conditions, complete conversion to the compound of formula (III) is routinely achieved in 2 to 6 hours.

The reaction may also be conducted in the presence of a catalyst, preferably an iodide salt and, preferably, in the presence of a catalyst and an antioxidant compound, as discussed hereinabove.

Upon complete conversion to the compound of formula (III), the compound of formula (IV), further inorganic base such as potassium carbonate and further solvent such as NMP is added to the same reaction vessel at a temperature of between 70°C and 80°C.

Preferably, following the addition of these reagents, the reaction mixture is allowed to cool from a temperature between 70°C and 80°C, to a temperature of between 60°C and 70°C, and the reaction mixture is allowed to stir.

Under these conditions, complete conversion to (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is routinely achieved in 8 to 12 hours.

The solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate may then be isolated by the process as described herein.

The present invention also provides the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained by the processes as described herein.

In one embodiment, the present invention provides the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained by the processes as described herein.

The present invention also provides the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as defined and characterised herein, obtained by the processes as described herein.

In particular, the invention provides the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained by the process of:
(i) dissolving (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate in dichloromethane to form a solution;
(ii) adding methyl tert-butyl ether to the solution; and
(iii) collecting the resulting precipitate from the solution.

The invention also provides the quasi-amorphous form of (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained by the process of:
(i) dissolving *(R)-tert-Butyl* (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate in dichloromethane to form a solution;
(ii) adding methyl tert-butyl ether to the solution; and
(iii) collecting the resulting precipitate from the solution.

As described herein, the process provides a solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate with beneficial physical characteristics that allow for isolation of an important intermediate in the synthesis of linagliptin, with a high yield and high purity, effectively in the absence of intermediate byproducts and impurities.

Accordingly, the present invention also provides the use of the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate for the preparation of linagliptin or a salt thereof.

In one embodiment, the invention provides the use of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate for the preparation of linagliptin or a salt thereof.

The present invention also provides the use of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as defined and characterised herein, for the preparation of linagliptin or a salt thereof.

Accordingly, the present invention also provides the use of the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate prepared by the processes as described herein, for the preparation of linagliptin or a salt thereof.

In one embodiment, the invention provides the use of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate prepared by the processes as described herein, for the preparation of linagliptin or a salt thereof.

The present invention also provides the use of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as defined and characterised herein and as prepared by the processes as described herein, for the preparation of linagliptin or a salt thereof.

To prepare linagliptin, the tert-butyloxycarbonyl protecting group is simply removed from (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

The skilled person would be aware from their common general knowledge of various procedures by which this protecting group could be removed.

Preferably, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is reacted with an acid to prepare linagliptin or a salt thereof.

Again, the skilled person would be aware from their common general knowledge of various acids and acidic conditions that would be suitable to remove the tert-butyloxycarbonyl protecting group to prepare linagliptin.

Preferably, sulfuric, hydrochloric or trifluoroacetic acid are used to remove the tert-butyloxycarbonyl protecting group.

The present invention also includes the use of the quasi-amorphous form of *(R)*-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as defined and characterised herein and as prepared by the processes as described herein, for the preparation of linagliptin dosage form, comprising the step of combining linagliptin with one or more pharmaceutically acceptable excipients to provide a linagliptin dosage form.

Linagliptin is typically administered orally and so the dosage form is preferably an oral dosage form, most preferably a tablet. In such a case, the process further comprises a tableting step. The oral dosage form may be a coated or uncoated tablet and may be prepared using standard techniques known in the art.

The dose of linagliptin is normally 5 mg given orally once daily. When linagliptin is added to metformin, the dose of metformin should be maintained, and linagliptin administered concomitantly.

Other preferred embodiments of the invention include:
1. A process for preparing linagliptin or a salt thereof, comprising (a) reacting a compound of formula (I) with a compound of formula (II) to prepare a compound of formula (III): 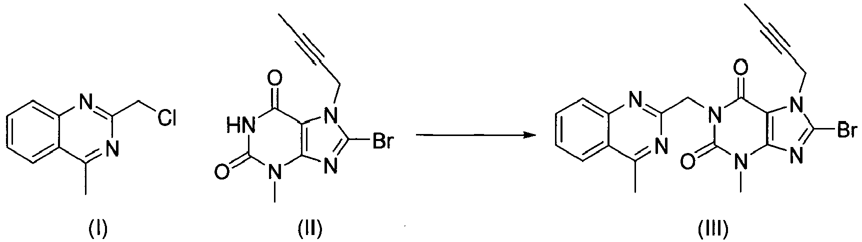
   wherein the reaction is performed in the presence of a catalyst and an antioxidant compound; and
   (b) converting the compound of formula (III) into linagliptin.
2. The process according to embodiment 1, wherein the catalyst is an iodide salt.
3. The process according to embodiment 1 or 2, wherein the catalyst is selected from potassium iodide, sodium iodide, lithium iodide or ammonium iodide.
4. The process according to any one of embodiments 1 to 3, wherein the antioxidant compound is selected from butylated hydroxyanisole, butylated hydroxytoluene, methyl gallate, ethyl gallate, propyl gallate, gallic acid, butylated hydroquinone and phenol.
5. The process according to any one of embodiments 1 to 4, wherein step (a) is performed in a nitrogen-containing solvent, preferably an N-alkylpyrrolidinone, dimethylformamide, or dimethylacetamide solvent.
6. The process according to any one of embodiments 1 to 5, wherein steps (a) and (b) are performed in one-pot.
7. A process for preparing linagliptin or a salt thereof, comprising the steps of (a) reacting a compound of formula (I) with a compound of formula (II) to prepare a compound of formula (III): 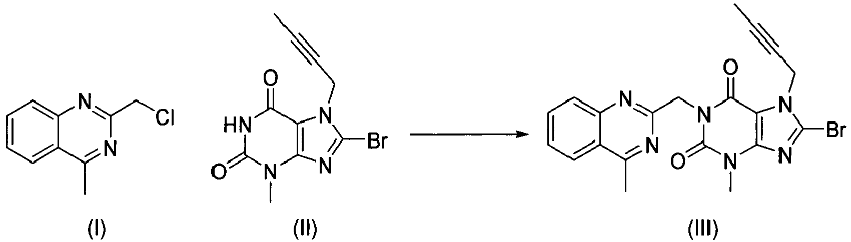
   wherein the reaction is performed in the presence of a catalyst and an antioxidant compound;
   (b) reacting the compound of formula (III) with a compound of formula (IV), or a salt thereof, to prepare a compound of formula (V); 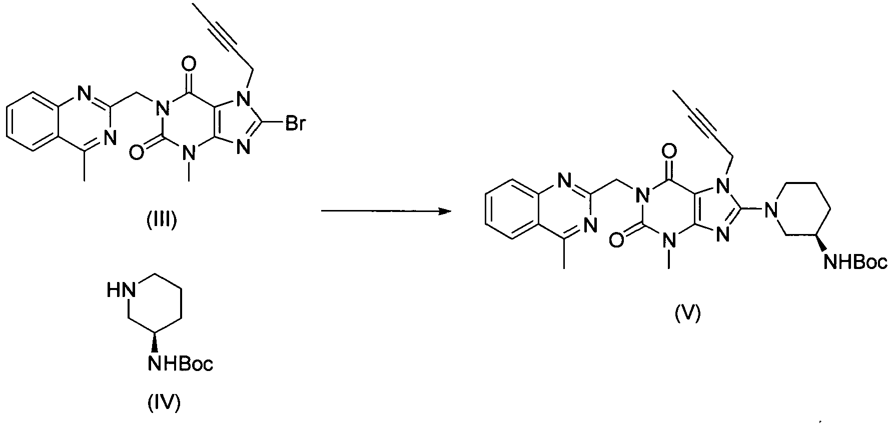
   and, (c) converting the compound of formula (V) into linagliptin.
8. The process according to embodiment 7, wherein the catalyst is an iodide salt.
9. The process according to embodiment 7 or 8, wherein the catalyst is selected from potassium iodide, sodium iodide, lithium iodide or ammonium iodide.
10. The process according to any one of embodiments 7 to 9, wherein the antioxidant compound is selected from butylated hydroxyanisole, butylated hydroxytoluene, methyl gallate, ethyl gallate, propyl gallate, gallic acid, butylated hydroquinone and phenol.
11. The process according to any one of embodiments 7 to 10, wherein step (a) is performed in a nitrogen-containing solvent, preferably an N-alkylpyrrolidinone, dimethylformamide, or dimethylacetamide solvent.
12. The process according to any one of embodiments 7 to 11, wherein steps (a) and (b) are performed in one-pot, optionally wherein steps (a), (b) and (c) are performed in one-pot.
13. Linagliptin or a salt thereof obtained by the process of any one of embodiments 1 to 6, or linagliptin or a salt thereof obtained by the process of any one of embodiments 7 to 12.
14. The use of linagliptin or a salt thereof prepared by the process of any one of embodiments 1 to 6, or any one of embodiments 7-12, for the preparation of a linagliptin dosage form.
15. The use of linagliptin or a salt thereof of embodiment 13 for the preparation of a linagliptin dosage form.

In these embodiments, the invention relates to a process for preparing linagliptin or a salt thereof, comprising (a) reacting a compound of formula (I) with a compound of formula (II) to prepare a compound of formula (III): 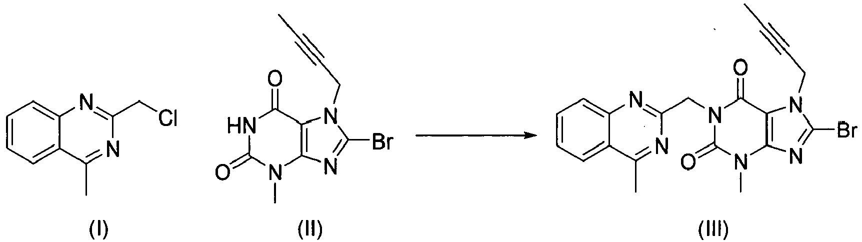
wherein the reaction is performed in the presence of a catalyst and an antioxidant compound; and
(b) converting the compound of formula (III) into linagliptin.

The invention also relates to a process for preparing linagliptin or a salt thereof, comprising the steps of (a) reacting a compound of formula (I) with a compound of formula (II) to prepare a compound of formula (III): 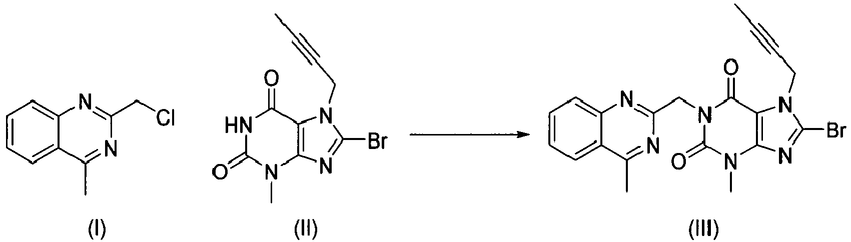
wherein the reaction is performed in the presence of a catalyst and an antioxidant compound;
(b) reacting the compound of formula (III) with a compound of formula (IV), or a salt thereof, to prepare a compound of formula (V); 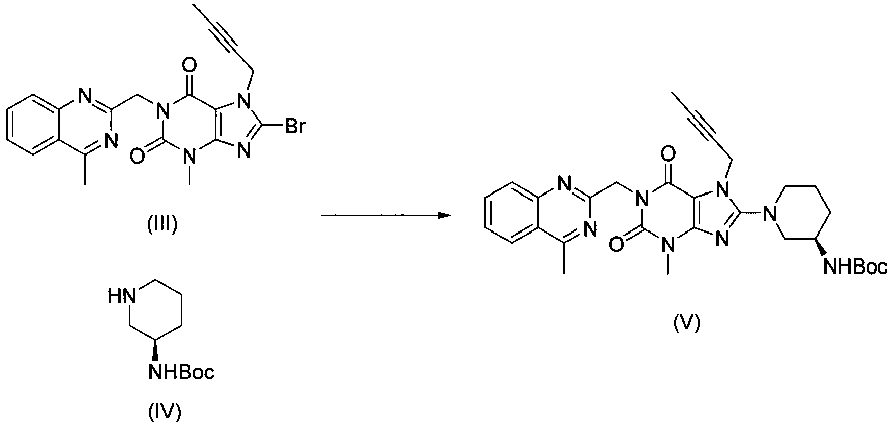
and, (c) converting the compound of formula (V) into linagliptin.

For these processes, the catalyst is preferably an iodide salt, for example potassium iodide, sodium iodide, lithium iodide or ammonium iodide. Most preferably, the catalyst is potassium iodide.

Equally, the antioxidant compound may be selected from butylated hydroxyanisole, butylated hydroxytoluene, methyl gallate, ethyl gallate, propyl gallate, gallic acid, butylated hydroquinone and phenol.

Preferably, the antioxidant compound is butylated hydroxyanisole.

Typically, the compound of formula (I) is reacted with the compound of formula (II) in the presence of a base, for example an inorganic base such as potassium carbonate (K₂CO₃).

The temperature of the reaction may vary depending upon the solvent used. Preferably, the reaction is conducted using a nitrogen-containing solvent as the reaction solvent, and the reaction is heated to a temperature of between 70°C and 80°C.

The nitrogen-containing solvent is typically an N-alkylpyrrolidinone, dimethylformamide, or dimethylacetamide.

Preferably, the nitrogen-containing solvent is N-methyl-2-pyrrolidone (NMP).

Under these conditions, complete conversion to compound of formula (III) is routinely achieved in 2 to 6 hours.

It is understood that a catalyst, in particular an iodide salt, advantageously increases the rate of reaction in the direction compound (III).

However, it is also understood that iodide salts may react with the reaction solvent, in particular if the reaction solvent is a nitrogen-containing solvent, such as NMP.

The inventors surprisingly discovered that if the reaction is conducted in the presence of an iodide salt catalyst and an antioxidant compound, then the reaction between the iodide salt and the nitrogen-containing solvent can be supressed or eliminated.

This is particularly advantageous, because the reaction is allowed to proceed in the presence of a catalyst and the formation of byproducts is reduced.

Thus, the inventors surprisingly discovered that by using a catalyst and an antioxidant compound, the yield and purity of compound (III) is increased, relative to the process absent an antioxidant compound. Consequently, because compound (III) (8-bromo-7-(but-2-yn-1-yl)-3-methyl-1-((4-methylquinazolin-2-yl) methyl)-3,7-dihydro-1H-purine-2,6-dione) is a key intermediate to provide linagliptin, this synthetic step can be advantageously used as part of a simplified synthesis to prepare linagliptin with an increased purity and/or yield.

In particular, the process of preparing the compound of formula (III) in the presence of a catalyst and an antioxidant renders one-pot processes feasible in terms of the overall yield and purity of the product obtained.

Therefore, steps (a) and (b) of the process as described in embodiments 1 to 5 may be performed in one-pot.

Equally, steps (a) and (b) or steps (a), (b) and (c) of the process as described in embodiments 7 to 11 may be performed in one-pot.

To prepare linagliptin, the tert-butyloxycarbonyl protecting group is simply removed from (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (compound V).

The skilled person would be aware from their common general knowledge of various procedures by which this protecting group could be removed.

Preferably, (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is reacted with an acid to prepare linagliptin or a salt thereof.

Again, the skilled person would be aware from their common general knowledge of various acids and acidic conditions that would be suitable to remove the tert-butyloxycarbonyl protecting group to prepare linagliptin.

Preferably, sulfuric, hydrochloric or trifluoroacetic acid are used to remove the tert-butyloxycarbonyl protecting group.

Linagliptin or a salt thereof may also be obtained by the process of any one of embodiments 1 to 6, or by the process of any one of embodiments 7 to 12.

Linagliptin or a salt thereof prepared by the process of any one of embodiments 1 to 6, or any one of embodiments 7-12, may also be used for the preparation of a linagliptin dosage form.

Linagliptin or a salt thereof of embodiment 13 may also be used for the preparation of a linagliptin dosage form.

The present invention will now be described with reference to the following examples, which are not intended to be limiting.

### Examples

### General methods

The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate was characterised as follows.

The sample was ground and XRPD was performed. The spectrum is shown in Fig. 1. The XRPD spectra were obtained using a Rigaku MiniFlex 600 X-Ray Diffractometer (Bragg-Brentano geometry) with Cu-Kα radiation (1.5406 Å) within the angle range of 2 to 40° 2θ with 0.01° step and scan speed of 6.0 deg/min. Limiting slit was adjusted to 10mm and Kβ filter was used at all times.

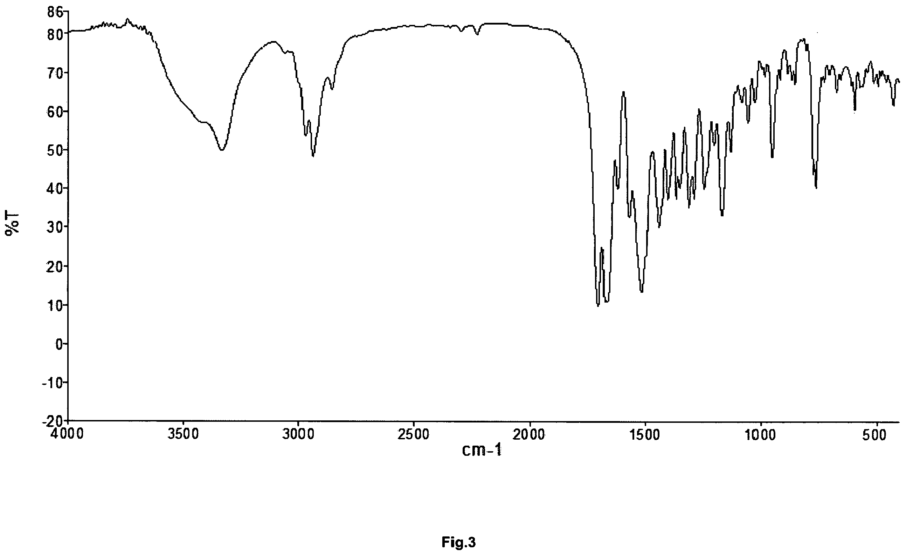

Differential scanning calorimetry (DSC) and infrared spectroscopy were also used and the DSC trace and IR spectrum are shown in Figs 2 and 3, respectively.

DSC was performed using a Mettler-Toledo Star DSC1 apparatus at a heating rate of 2.5°C/min. The DSC onset was 144.84°C; the peak 154.88°C; and the endset 157.10°C.

The IR spectra were obtained according to Ph.Eur. (2.2.24.) and USP, <197K> using the KBr disc method. The apparatus was Perkin Elmer's SPECTRUM TWO.

The IR peak listing are at (cm⁻¹): 3333.95; 2942.79; 1701.86; 1664.74; 1621.76; 1569.08; 1515.67; 1439.86; 1401.37; 1365.81; 1311.49; 1288.48; 1245.09; 1167.28; 1129.38; 1054.48; 953.96; 761.35; 595.61.

The purity of the (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained was assessed by UPLC (ultra performance liquid chromatography) with UV detector according to the following protocol.

### Column:

- Acquity UPLC BEH C18 2.1 x 100mm; particle size 1.7 µm (Waters, cat. no. 186002352);

### Mobile phase:

- mobile phase A: - 0.1% orthophosphoric acid in water (add 1.0 mL of orthophosphoric acid, 85% into 1000 mL of purified water, mix and degas using ultrasonic bath);
- mobile phase B: acetonitrile;

Flow rate: 0.3 mL/min.
Detection: spectrophotometer at 228 nm.
Injection: 1.0 µL of the test solution.

### Standard solution I:

Weigh accurately about 20.0 mg of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (compound V) standard substance into a 100 mL volumetric flask, dissolve in diluent using ultrasonic bath and fill up to the volume with diluent.

### Standard solution II:

Weigh accurately about 20.0 mg of 8-bromo-7-(but-2-yn-1-yl)-3-methyl-1-((4-methylquinazolin-2-yl) methyl)-3,7-dihydro-1H-purine-2,6-dione (compound III) standard substance into a 100 mL volumetric flask, dissolve in diluent using ultrasonic bath and fill up to the volume with diluent.

### Standard solution III:

Transfer 1.0 mL of standard solution I and 1.0 mL of standard solution II into a 50 mL volumetric flask, fill up to the volume with diluent.

### Standard solution:

Transfer 1.0 mL of standard solution III into a 20 mL volumetric flask and fill up to the volume with diluent (compound III, 0.10%, and compound V, 0.10%).

### Test solution I:

Weigh accurately about 20.0 mg of test substance into a 100 mL volumetric flask, dissolve in diluent and fill up to the volume with diluent. (0.2 mg/mL).

### Test solution II:

Transfer 1.0 mL of test solution I into a 50 mL volumetric flask and fill up to the volume with diluent.

### Column conditioning:

Start column conditioning by setting up flow rate of mobile phase A : mobile phase B (90:10; v/v) at 0.20 mL/min for 10 minutes at temperature of 40°C and gradually increase the flow rate up to 0.30 mL/min. Hold the flow rate until backpressure is stable. Monitor until the baseline is stable.

The analytical method is based on gradient of the mobile phase, thus 2 chromatographic analyses without the injection (condition column) and several injections of diluent were performed.

### Procedure:

After equilibration of chromatographic system perform analyses of the diluent, standard solution, standard solution III, test solution II and test solution I were performed, the respective chromatograms recorded, and the purity of the substances contained within the test solutions was calculated accordingly.

The retention time of *R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate according to this protocol is about 7.1 minutes.

### Example 1: Preparation of (R)-tert-Butyl (1-(7-(but-2-vn-1-vl)-3-methyl-1-((4-methylquinazolin-2-yl)methyl)-2,6-dioxo-2,3,6,7-tetrahydro-1H-purin-8-yl) piperidin-3-vl) carbamate (compound V)

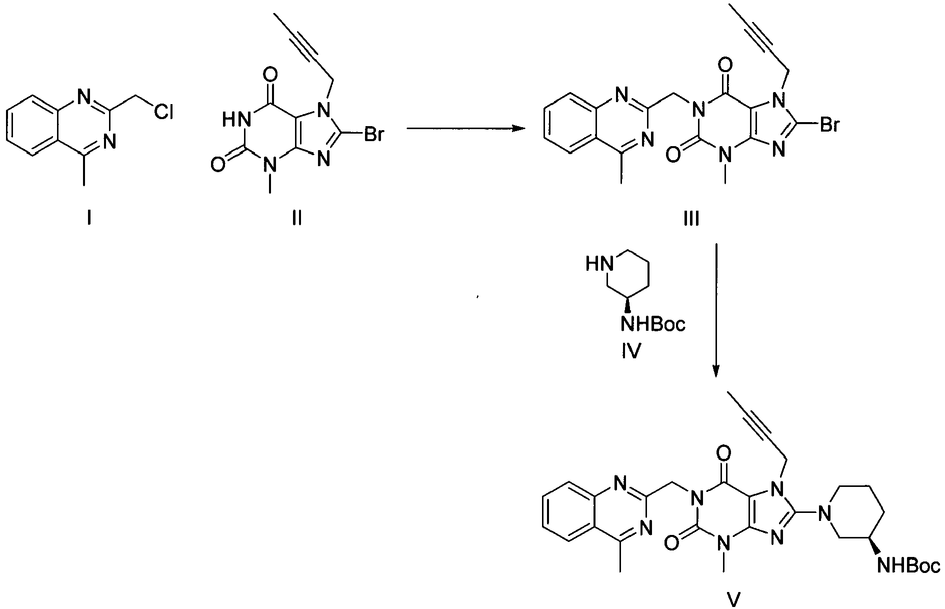

2-(Chloromethyl)-4-methyl-quinazoline (compound I) (66.13 g, 0.343 mol) and 3-methyl-7-(2-butyn-1-yl)-8-bromo-xanthine (compound II) (100.00 g, 0.337 mol), potassium carbonate (53.50 g, 0.387 mol), potassium iodide (2.79 g, 0.017 mol) and *N*-methyl-2-pyrrolidone (450 mL) were charged into a reactor. Shortly after stirring was commenced, reaction mixture was heated to 75-80°C and stirred for 4 hours. After this time, the reaction should have proceeded to completion, to obtain 8-bromo-7-(but-2-yn-1-yl)-3-methyl-1-((4-methylquinazolin-2-yl) methyl)-3,7-dihydro-1H-purine-2,6-dione (compound III).

Compound (IV) (77.52 g, 0.387 mol), potassium carbonate (53.50 g, 0.387 mol) and N-methyl-2-pyrrolidone (50 mL) were then charged directly to the reactor containing compound III. After the addition was complete, the reaction mixture was allowed to cool to 60-65°C and stirred for 10 hours.

After reaction completion, toluene (750 mL) was added to the mixture and the resulting suspension was stirred for 30 minutes. Next, water (500 mL) was added dropwise to the suspension over a period of 1 hour, and then the mixture was stirred for additional 1 hour at 80-85°C. After this time, stirring was stopped and the layers were separated. The toluene layer was collected, and the water layer was extracted using toluene (500 mL). The toluene layers were combined, transferred into the reactor, and washed with a 10% brine solution (500 mL) and subsequently, with water (500 mL). Then, the toluene layer was collected and evaporated until dryness. Dichloromethane (DCM) (100 mL) was then added to the dry residue, and then removed via evaporation.

Crude (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate residue was then dissolved in dichloromethane (300 mL) at 30°C. To this solution, activated charcoal (20.0 g) and magnesium sulfate (30.0 g) was charged. The resulting suspension was stirred for 2 hours and then the solids were removed by filtration. The filtrate was then heated to 35°C and methyl tert-butyl ether (MTBE) (1500 mL) was added dropwise thereto. Upon complete addition of the methyl tert-butyl ether, the solution was cooled to 5°C at a rate of -10°C/hour and stirred at this temperature for an additional 2 hours.

The resulting precipitate was filtered, washed with DCM:MTBE, 1:4 solution, (200 mL) and then dried in vacuum drier at 50°C/20mbar, to provide the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

144.66 g of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate was obtained (75.0% yield from compound II, with a purity of 97.5-98% as determined by UPLC).

### Example 2: Preparation of (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-vl) carbamate (compound V)

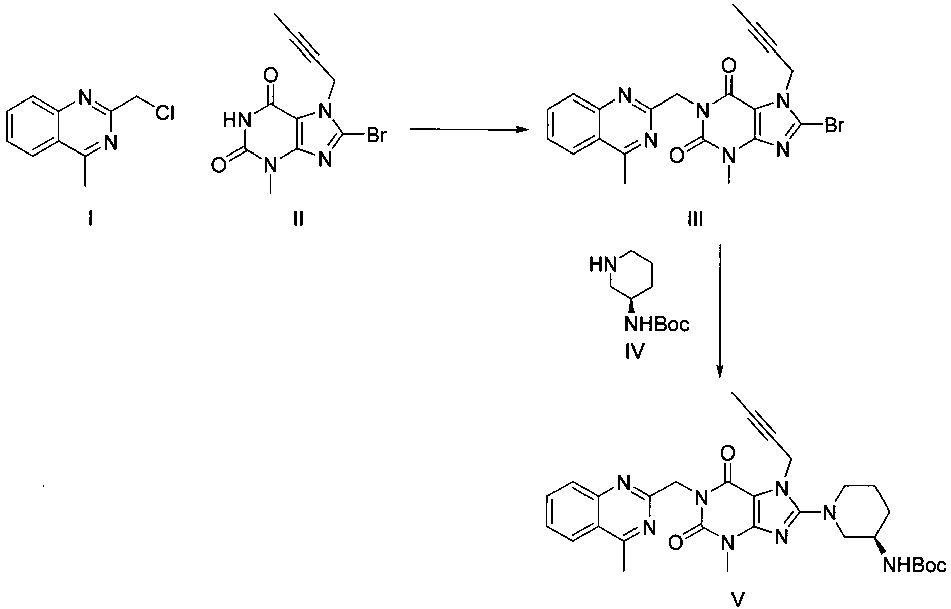

2-(Chloromethyl)-4-methyl-quinazoline (compound I) (66.13 g, 0.343 mol) and 3-methyl-7-(2-butyn-1-yl)-8-bromo-xanthine (compound II) (100.00 g, 0.337 mol), potassium carbonate (53.50 g, 0.387 mol), potassium iodide (2.79 g, 0.017 mol), butylated hydroxyanisole (3.03 g, 0.017 mol) and *N*-methyl-2-pyrrolidone (450 mL) were charged into a reactor. Shortly after stirring was commenced, reaction mixture was heated to 75-80°C and stirred for 4 hours. After this time, the reaction should have proceeded to completion, to obtain 8-bromo-7-(but-2-yn-1-yl)-3-methyl-1-((4-methylquinazolin-2-yl) methyl)-3,7-dihydro-1H-purine-2,6-dione (compound III).

Compound (IV) (77.52 g, 0.387 mol), potassium carbonate (53.50 g, 0.387 mol) and *N*-methyl-2-pyrrolidone (50 mL) were then charged directly to the reactor containing compound III. After the addition was complete, the reaction mixture was allowed to cool to 60-65°C and stirred for 10 hours.

After reaction completion, toluene (750 mL) was added to the mixture and the resulting suspension was stirred for 30 minutes. Next, water (500 mL) was added dropwise to the suspension over a period of 1 hour, and then the mixture was stirred for additional 1 hour at 80-85°C. After this time, stirring was stopped and the layers were separated. The toluene layer was collected, and the water layer was extracted using toluene (500 mL). The toluene layers were combined, transferred into the reactor, and washed with a 10% brine solution (500 mL) and subsequently, with water (500 mL). Then, the toluene layer was collected and evaporated until dryness. Dichloromethane (DCM) (100 mL) was then added to the dry residue, and then removed via evaporation.

Crude (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate residue was then dissolved in dichloromethane (300 mL) at 30°C. To this solution, activated charcoal (20.0 g) and magnesium sulfate (30.0 g) was charged. The resulting suspension was stirred for 2 hours and then the solids were removed by filtration. The filtrate was then heated to 35°C and methyl tert-butyl ether (MTBE) (1500 mL) was added dropwise thereto. Upon complete addition of the methyl tert-butyl ether, the solution was cooled to 5°C at a rate of -10°C/hour and stirred at this temperature for an additional 2 hours.

The resulting precipitate was filtered, washed with DCM:MTBE, 1:4 solution, (200 mL) and then dried in vacuum drier at 50°C/20mbar, to provide the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

165.42 g of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate was obtained (85.8% yield from compound II, with a purity of 98.5-99% as determined by UPLC).

### Example 3: Preparation of (R)-tert-Butyl (1-(7-(but-2-vn-1-yl)-3-methyl-1-((4-methylquinazolin-2-yl)methyl)-2,6-dioxo-2,3,6,7-tetrahydro-1H-purin-8-vl) piperidin-3-vl) carbamate (compound V)

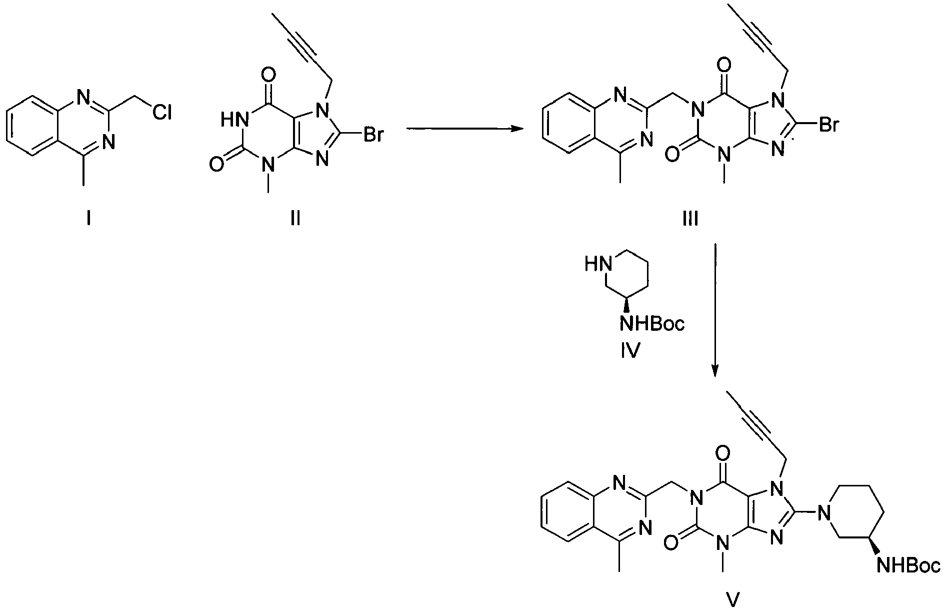

2-(Chloromethyl)-4-methyl-quinazoline (compound I) (66.13 g, 0.343 mol) and 3-methyl-7-(2-butyn-1-yl)-8-bromo-xanthine (compound II) (100.00 g, 0.337 mol), potassium carbonate (53.50 g, 0.387 mol), potassium iodide (2.79 g, 0.017 mol), butylated hydroxyanisole (3.03 g, 0.017 mol) and *N*-methyl-2-pyrrolidone (450 mL) were charged into a reactor. Shortly after stirring was commenced, reaction mixture was heated to 75-80°C and stirred for 4 hours. After this time, the reaction should have proceeded to completion, to obtain 8-bromo-7-(but-2-yn-1-yl)-3-methyl-1-((4-methylquinazolin-2-yl) methyl)-3,7-dihydro-1H-purine-2,6-dione (compound III).

Compound (IV) (77.52 g, 0.387 mol), potassium carbonate (53.50 g, 0.387 mol) and *N*-methyl-2-pyrrolidone (50 mL) were then charged directly to the reactor containing compound III. After the addition was complete, the reaction mixture was allowed to cool to 60-65°C and stirred for 10 hours.

After reaction completion, toluene (750 mL) was added to the mixture and the resulting suspension was stirred for 30 minutes. Next, water (500 mL) was added dropwise to the suspension over a period of 1 hour, and then the mixture was stirred for additional 1 hour at 80-85°C. After this time, stirring was stopped and the layers were separated. The toluene layer was collected, and the water layer was extracted using toluene (500 mL). The toluene layers were combined, transferred into the reactor, and washed with a 10% brine solution (500 mL) and subsequently, with water (500 mL). Then, the toluene layer was collected and evaporated until dryness. Dichloromethane (DCM) (100 mL) was then added to the dry residue, and then removed via evaporation.

Crude (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate residue was then dissolved in dichloromethane (300 mL) at 30°C. To this solution, activated charcoal (20.0 g) and magnesium sulphate (30.0 g) was charged. The resulting suspension was stirred for 2 hours and then the solids were removed by filtration. The filtrate was then heated to 35°C and all volatiles were removed by evaporation under reduced pressure, and the obtained product was finally dried in vacuum drier at 50°C/20mbar.

165.42 g of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate was obtained (85.8% yield from compound II, with a purity of 96% as determined by UPLC).

### Example 4: Preparation of 8-[(3R)-3-aminopiperidin-1-yl]-7-but-2-ynyl-3-methyl-1-[(4-methylquinazolin-2-vl)methyllpurine-2,6-dione (linaqliptin)

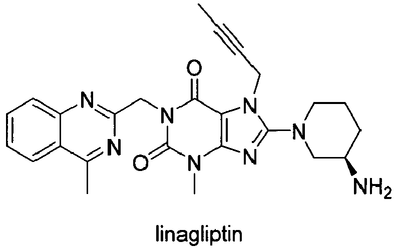

(*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate (100.00 g, 0.175 mol), MeOH (1000 mL), water (500 mL) and 98% sulfuric acid (46.80 mL, 85.64g, 0.87 mol) were charged into a reactor. After a thorough mixing at room temperature, the reaction mixture was heated to 60°C, stirred until dissolution and then stirred for 4 hours. Then, the MeOH/water phase was cooled to 30°C, the pH was adjusted to 11.5-12.5 with a 30% NaOH solution, and the mixture was stirred for additional 2 hours. Next, the resulting suspension was filtered, the filtrate was collected, combined with dichloromethane (500 mL) and 10% brine solution (500 mL), and then transferred to the reactor, whilst the reaction mixture remained at 30°C. After stirring for 0.5 hour, the phases were left to separate, the dichloromethane layer was collected and the water layer was extracted with dichloromethane (250 mL). The water layer was then discarded, while the collected organic layers were combined and washed with a 10% brine solution (500 mL) and subsequently, with water (500 mL). Water (1000 mL) and acetic acid (40 mL) was then added to the dichloromethane layers, stirred for 0.5 hours and then left to separate. The water layer was left in the reactor and washed twice with dichloromethane (250 mL). After washing, dichloromethane (500 mL) was added, pH was again corrected to 11.5 - 12.5, and the mixture was stirred for additional 0.5 hours, after which phases were left to separate and dichloromethane layer was collected. The water layer was re-extracted with dichloromethane (250 mL), which was again separated and combined with previous dichloromethane layer. Combined dichloromethane solutions were washed twice with water (500 mL). All volatiles were then evaporated by distillation (40°C) followed by vacuum distillation (up to 60°C/20mbar).

To the resulting dry residue, EtOH (300 mL) and MTBE (25 mL) were added, and the mixture was heated to 50°C and stirred until full dissolution. MTBE (1200 mL) was then added dropwise to the solution and resulting crystalline suspension was stirred for 1 hour at 50°C, before being cooled to -10°C, at a rate of -10°C/hour. The mixture was then stirred at this temperature for an additional 2 hours. The resulting precipitate was filtered, washed with EtOH:MTBE, 1:4 solution, (100 mL) and then dried in vacuum drier at 35°C/20mbar, of which 72.4 g (89.9%) of crude linagliptin was obtained.

### Example 5: Recrystallisation of 8-[(3R)-3-aminopiperidin-1-yl]-7-but-2-ynyl-3-methyl-1-[(4-methyl quinazolin-2-yl)methyl]purine-2,6-dione (linagliptin)

Linagliptin (50.00 g, 0.106 mol) was charged into a reactor followed by EtOH (150 mL) and MTBE (12.5 mL), and the mixture was heated to 50°C and stirred until dissolution. MTBE (600 mL) was then added dropwise to the solution and resulting crystalline suspension was stirred for 1 hour at 50°C, before being cooled to -10°C, at a rate of -10°C/hour. The mixture was then stirred at this temperature for an additional 2 hours. The resulting precipitate was filtered, washed with EtOH:MTBE, 1:4 solution, (50 mL) and then dried in vacuum drier at 35°C/20mbar, of which 45.5 g (91.0%) of Linagliptin was obtained.

## Claims

1. A quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate.

2. The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as claimed in claim 1, **characterised by** XRPD peaks at 2θ (°) ± 0.2°: 3.0, 10.3, 15.3, 18.8, 21.4 and 26.5.

3. The quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as claimed in claim 1 or claim 2, **characterised by** broad XRPD peaks, wherein the breadth of each peak, at the base of each peak is 2θ (°) ± 0.2°: 2.3-4.0, 9.7-10.7, 14.8-15.8, 20.5-22.5 and 25.8-27.5.

4. The quasi-amorphous form of (*R*)-(1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as claimed in any one of claims 1 to 3, **characterised by** a differential scanning calorimetry (DSC) thermogram having a melting onset at 144-146°C, a melting endset at 156-158°C and a melting point peak at 154-156°C.

5. A process for the preparation of a solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate comprising:
(i) dissolving *(R)-tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate in dichloromethane to form a solution;
(ii) adding methyl tert-butyl ether to the solution; and
(iii) collecting the resulting precipitate from the solution.

6. The process as claimed in claim 5, wherein (R)-tert-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is prepared by reacting a compound of formula (III) with a compound of formula (IV), or a salt thereof: 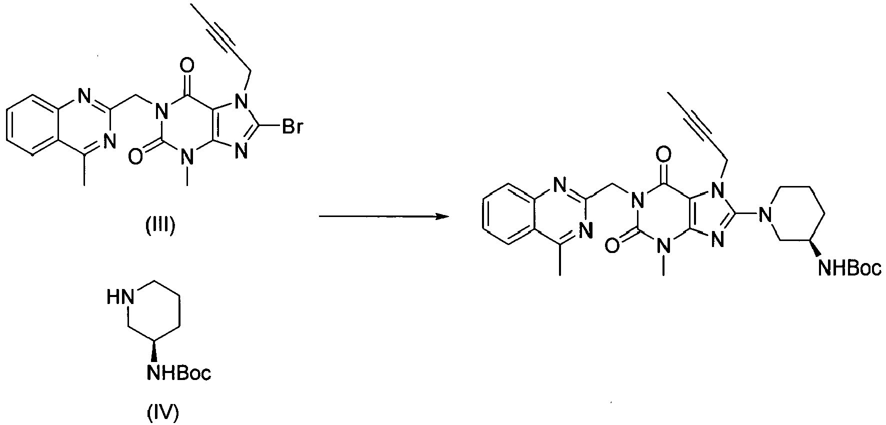 prior to steps (i) to (iii).

7. The process as claimed in claim 6, wherein the compound of formula (III) is prepared by reacting a compound of formula (I) with a compound of formula (II):

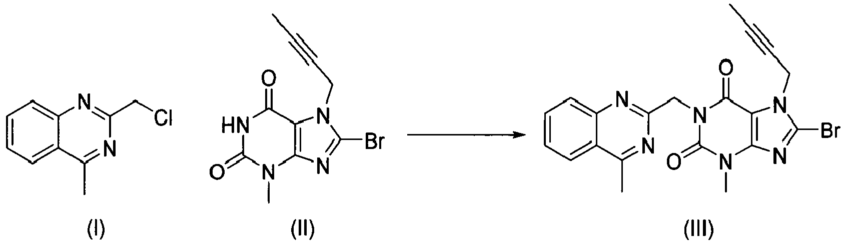

8. The process as claimed in any one of claims 5 to 7, wherein (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is prepared according to the following process: 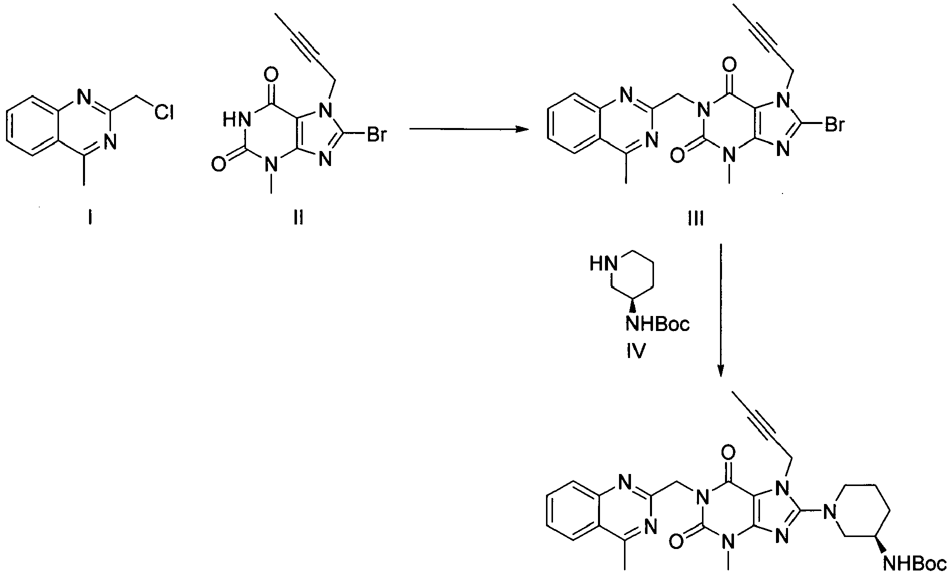 wherein the process is a one-pot process.

9. The process as claimed in claim 7 or 8, wherein the compound of formula (III) is prepared by reacting a compound of formula (I) with a compound of formula (II) in the presence of a catalyst, preferably in the presence of a catalyst and an antioxidant compound.

10. The process as claimed in claim 9, wherein the antioxidant compound is selected from butylated hydroxyanisole, butylated hydroxytoluene, methyl gallate, ethyl gallate, propyl gallate, gallic acid, butylated hydroquinone and phenol.

11. The process as claimed in any one of claims 5 to 10 wherein the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate is the quasi-amorphous form as claimed in any one of claims 1 to 4.

12. The solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate obtained by the process as claimed in any one of claims 5 to 10.

13. The use of the quasi-amorphous form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as claimed in any one of claims 1 to 4, or the use of the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate as claimed in claim 12, for the preparation of linagliptin or a salt thereof.

14. The use of the solid form of (*R*)-*tert*-Butyl (1-(7-(but-2-yn-1-yl)-3-methyl-1-((4-methylquinazolin-2-yl)methyl)-2,6-dioxo-2,3,6,7-tetrahydro-1H-purin-8-yl) piperidin-3-yl) carbamate prepared by the process as claimed in any one of claims 5 to 11, for the preparation of linagliptin or a salt thereof.

15. The use as claimed in claims 13 or 14, comprising the step of combining linagliptin with one or more pharmaceutically acceptable excipients to provide a linagliptin dosage form.
